(19) Europäisches Patentamt
European Patent Office
Office européen des brevets

(11) Publication number: **0 549 023 A2**

# (12) EUROPEAN PATENT APPLICATION

(21) Application number: **92203772.6**

(22) Date of filing: **04.12.92**

(51) Int. Cl.5: **C07C 69/76**, C08K 5/12, C08K 5/37, C08K 5/42

(30) Priority: **24.12.91 GB 9127588**

(43) Date of publication of application: **30.06.93 Bulletin 93/26**

(84) Designated Contracting States: **DE FR GB**

(71) Applicant: **FMC CORPORATION (UK) LIMITED**
**Tennax Road, Trafford Park**
**Manchester M17 1WT(GB)**

(72) Inventor: **Schneider, Michael James**
**6 Deansway, Higher Kinnerton**
**Near Chester CH4 9DE(GB)**
Inventor: **Gainer, James**
**7 Stechworth Drive**
**Boothstown, Worsley M28 4FU(GB)**

(74) Representative: **W.P. Thompson & Co.**
**Coopers Building, Church Street**
**Liverpool L1 3AB (GB)**

## (54) Flame retardant compounds.

(57) The invention provides flame retardant esters of the formula I

Xn - Ar - [CO2CH2C(CH2Br)3]m (I)

where Ar represents phenyl, naphthyl, or two aryl rings either directly connected or linked via $-CH_2-$.

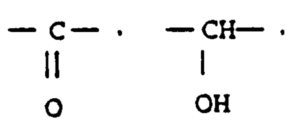

-O-.

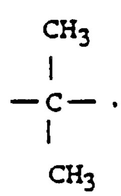

-CH=CH-. -CHBr- CHBr-. -S-. or

EP 0 549 023 A2

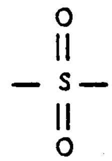

X is Cl or Br, preferably Br; m is 3 or 4; and n is 0 to 6; there being two of the ester groups in the ortho position to each other.

This invention relates to flame retardant compounds and compositions containing them.

Brominated organic compounds are well known to have flame retardant properties and are extensively used for this purpose in a wide variety of materials such a plastics and rubber, and including cellular and foamed materials such as foamed polystyrene and foamed polyurethanes.

The vapor phase mode of action of these compounds relies to a great extent on the thermal stability of the brominated compound in relation to that of the polymer. If the flame retardant compound has too low a thermal stability, i.e. begins to decompose or vaporize at too low a temperature, then it will be exhausted before the polymer itself starts decomposing. If the flame retardant has too high a thermal stability, it will remain in the condensed phase, and will therefore, remain inactive as the polymer decomposes. Thus, it is desirable to have a flame retardant compound whose thermal stability closely matches that of the polymer.

The thermal stability of various known flame retardants is given below, the accepted measure of thermal stability being the temperature in °C at which there is a 10% loss in weight; ("Thermal Characteristics of Polymer Materials", E.A. Turi, Academic Press, 1981);

| | 10% wt. loss at |
|---|---|
| decabromodiphenyloxide (DBDPO) | 373°C |
| octabromodiphenyloxide (OBDPO) | 340°C |
| bis(2-hydroxyethyl)ether of tetrabromobisphenol A | 337°C |
| octabromodiphenyl oxide | 336°C |

Of these DBDPO has been extensively used a a flame retardant in ABS (acrylonitrile-butadiene-styrene) rubbers and other similar engineering thermoplastics (ETP) that have a high thermal stability, e.g. HIPS, and therefore, require a high thermal stability flame retardant to match. ABS, for example, shows decomposition to 10% weight loss at about 390°C (Stanton Redcroft STA 100,10°C $min.^{-1}$ 30 $ml.min.^{-1}$ air, sample weight 10±0.02 mg. Iconel crucible). This figure is reasonably closely matched by that of DBDPO at 373°C. However, the use of DBDPO has recently come into question, as it is suspected of forming highly toxic brominated dibenzodioxins and -furans upon pyrolysis, and these products have, indeed, been detected in the pyrolysis of polymer systems containing DBDPO.

Octabromodiphenyloxide has been substituted for DBDPO but is less effective as a flame retardant, and is also suspected of forming brominated dibenzo-dioxins and -furans upon pyrolysis.

Thus, a need still exists for flame retardant compounds of high thermal stability and which are not prone to the formation of such toxic substances upon pyrolysis.

In GB Patent No. 1,341,458, flame retardant benzene polycarboxylic esters of halogenated monohydric alcohols having a neopentyl-type configuration are disclosed, namely compounds of the formula

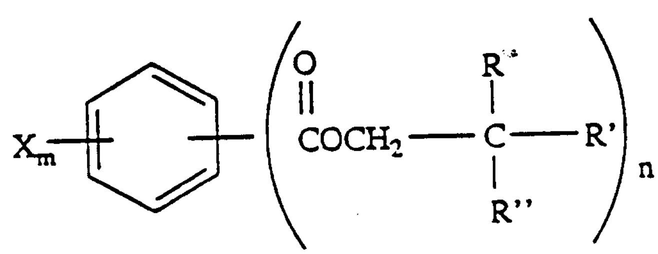

where R,R' and R" are halogenated methyl groups, X is halogen, n is 2 or 3, and m is 0-4, with the proviso that, when n is 2, the ester groups are in the meta- or para-postitions on the benzene ring, and when n is 3 (in which case m is correspondingly 0-3) the ester groups are in the 1,3 and 5 positions. In other words, the compounds disclosed in GB 1,341,458 are the halogenated neopentyl, especially the tribromoneopentyl esters of isophthalic acid, terephthalic acid and trimesic acid, and their halogenated derivatives.

In GB 1,341,458 attention is focused on esters of iso- and terephthalic acid and of trimesic acid having aliphatic, rather than aromatic, halogenation, that is to say wherein the bromine or chlorine substituents are in the aliphatic side chains rather than in the aromatic ring. Aromatic substitution is embraced within the broad formula, but only one working example is given, namely the preparation of the compound di[2,2-bis-(bromomethyl)-3-bromopropyl] tetrachloroterephthalate, or, in accordance with the trivial, rather than systematic, nomenclature used herein: bis-(tribromoneopentyl)tetrachloroterephthalate. There is no exemplification of any compounds having brominated aromatic rings.

The esters disclosed in GB 1,341,458 are indicated to have flame retardant properties, and to be useful a flame retardant additives in a wide range of synthetic plastics, *inter alia* hydrocarbon polymers such as polypropylene, polystyrene, styrene-butadiene rubber and butyl rubber, and non-hydrocarbon polymers, e.g. polyesters such as poly(ethylene terephthalate), polyurethanes, poly(alkylene oxides), ethylene-vinyl chloride) copolymers. Also included are references to butadienearylonitrile and butadiene-acrylonitrile-styrene copolymers, but no such formulations appear to have been manufactured or tested.

The compounds of GB 1,341,458 are thus put forward a general purpose flame retardants to be used in amounts of from 3 to 30% by weight based on the total weight of the composition in the case of chlorinated compounds, and from 1 to 15% by weight in the case of the brominated compounds, usually in combination with 1 to 15% by weight of any antimony compound such a antimony trioxide. The weight loss of the claimed compounds is said to be less than 5% at up to 300 °C, but with values ranging from 7 to 34% at 350 °C.

The preferred applications are indicated to be a flame retardants in homopolymers or random, block or graft copolymers of alpha-olefins of 2 to 6 carbon atoms, polystyrene, polyurethanes, polyamides and polyesters.

Various other tetrahalophthalate esters are known and have been proposed a flame retardant additives, particularly for polyphenylene ether resins. Reference may be made in this respect to WO 88/03542 which mentions the use *inter alia* of alkyl diesters of tetrabromophthalic acid for this purpose, and including a broad reference also to $C_2$-$C_6$ haloalkyl esters of tetrabromophthalic acid, although no specific examples are given. The preferred compounds are the alkyl diesters, specifically di-(2-ethylhexyl)tetrabromophthalate. Also mentioned is the di-(hydroxypropyl methoxy polyoxyethylene glycol) ester of tetra\bromophthalic acid.

Compounds of a similar structure are to be found in WO 87/01713 which discloses flame retardant tetrabromo- and tetrachloro-phthalate esters comprising the mono-, di- and polyesters of tetrabromo- and tetrachloro-phthalic acid and a polyalkyleneglycol or glycol ether, e.g. polyethylene glycol (carbowax), polypropylene glycol, or the corresponding monoethers. Thermal stability figures are not given, but these polyoxyalkylene tetrabromo- and tetrachloro-phthalate esters are said, in particular, to possess improved processability in polyphenylene ether resins and resin blends, e.g. blends with other EP resins, particularly vinyl aromatic resins such as polystyrene, styrene-acrylonitrile copolymers, styrene-butadiene copolymers and ABS.

In contrast to the foregoing, and other prior art, and in accordance with the present invention, the tribromoneopentyl esters of trimellitic acid and aromatic tetracarboxylic acids, and especially the ring brominated derivatives, have been found to be unexpectedly advantageous as flame retardants in comparison to other halogenated benzene di- and tricarboxylic acids, e.g. the haloneopentyl esters of tetrabromo- and tetrachloro-terephthalic and isophthalic acid disclosed in GB 1,341,458, and are especially useful as non-dioxin forming flame retardants in acrylonitrile-butadiene-styrene (ABS) rubbers, high impact polystyrene (HIPS) and other engineering plastics (EPs).

Accordingly, the present invention provides novel haloneopentyl esters of formula (I)

$$Xn - Ar - [CO_2CH_2C(CH_2Br)_3]m \qquad (I)$$

where Ar represents phenyl, naphthyl, or two aryl rings either directly connected or linked via -$CH_2$-.

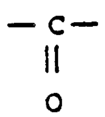
,
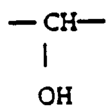
,

-O-.

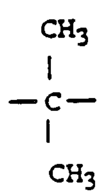
,

-CH = CH-. -CHBr-CHBr-. -S-. or

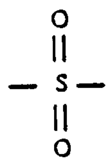

X is Cl or Br, preferably Br; m is 3 or 4; and n is 0 to 6; there being two of the ester groups in the ortho position to each other.

Preferred compounds of the invention are those where m is 4 and Ar is phenyl or biphenyl

Surprisingly, in view of steric considerations due to the close proximity of a many bromomethyl (-$CH_2Br$) groups in the end product, the novel compounds of this invention may be readily prepared in high yield by the reaction of tribromoneopentyl alcohol (TBNPA) with the appropriate acid or anhydride, e.g. pyromellitic dianhydride or hexabromodiphenyl-4,4-dianhydride, or the corresponding acid chlotides. Generally speaking the reaction is conducted under reflux conditions in a water immiscible solvent, such as toluene, with continuous removal of water, e.g. by Dean Stark trap, and in the presence of an esterification catalyst such as $H_2SO_4$, $H_3PO_4$ para-toluene sulphonic acid (PTSA) etc.

TBNPA Is obtained by the hydrobromination of pentaerythritol for example, a taught in US-A-3,932,541, US-A-3,954,874 and US-A-4,154,966. The acid reagents, for example, pyromellitic dianhydride or hexabromodiphenyl-3,3'-4,4'-dianhydride are either commercially available, or can readily be prepared by bromination or chlorination of the appropriate acid or anhydride.

Whilst this represents the presently preferred method, other routes are available, for example, transesterification reaction of TBNPA with the methyl ester of the desired acid or anhydride. Such esterification reactions are, however, conventional.

In the above formulae, the halogen atoms in the aliphatic side chain need not be the same as those in the aromatic nucleus. Thus the invention contemplates bromo-/chloro- compounds, with bromine in the side chain and chlorine in the aromatic nucleus, as well as the purely brominated compounds containing bromine in both the aromatic nucleus and the side chain.

The compounds of the invention are useful as flame retardants in a wide variety of polymers including:

1. Polystyrene, poly-(p-methylstyrene) or poly-($\alpha$-methylstyrene).

2. Copolymers of styrene or $\alpha$-methylstyrene with dienes or acrylic derivatives, such as, for example, styrene/butadiene, styrene/acrylonitrile, styrene/alkyl methacrylate, styrene/maleic anhydride, styrene/butadiene/ethyl acrylate, styrene/acrylonitrile/methyl acrylate; mixtures of high impact strength from styrene copolymers and another polymer, such as, for example, styrene/butadiene/styrene, styrene/isoprene/-styrene, styrene/ethylene/butylene/styrene or styrene/ethylene/propylene/styrene.

3. Graft copolymers of styrene or $\alpha$-methylstyrene such as, for example, styrene on polybutadiene, styrene on polybutadiene-styrene or polybutadiene-acrylonitrile; styrene and acrylonitrile (or methyacrylonitrile) on polybutadiene; styrene and maleic anhydride or maleimide on polybutadiene; styrene, acrylonitrile and maleic anhydride or maleimide on polybutadiene; styrene, acrylonitrile and methyl methacrylate on polybutadiene, styrene, acrylonitrile and methyl methacrylate on polybutadiene, styrene and acrylonitrile on ethylene/-propylene/diene terpolymers, styrene and acrylonitrile on polyacrylates or polymethacrylates, styrene and acrylonitrile on acrylate/butatdiene copolymers, as well as mixtures thereof with the copolymers listed under 5), for instance the copolymer mixtures known as ABS-, MBS-, ASA- or AES-polymers.

4. Polymers which are derived from $\alpha,\beta$-unsaturated acids and derivatives thereof, such as polyacrylates and polymethacrylates, polyacrylamide and polyacrylonitrile.

5. Copolymers from the monomers mentioned under 4), with each other or with other unsaturated monomers, such as, for instance, acrylonitrile/butadiene, acrylonitrile/alkyl acrylate, acrylonitrile/alkoxyalkyl acrylate or acrylonitrile/vinyl halogenide copolymers or acrylonitrile/alkyl methacrylate/butadiene terpolymers, or maleic anhydride/-styrene/methyl methacrylate copolymers.

6. Polyphenylene oxides and sulfides, and mixtures or blends of polyphenylene oxides with polystyrene, graft polymers or styrene copolymers e.g. high-impact strength polystyrene, and EPDM copolymers with rubbers, and mixtures of polyphenylene oxides with polyamides.

7. Polyurethanes which are derived from polyethers, polyesters or polybutadienes with terminal hydroxyl groups on the one side and aliphatic or aromatic polyisocyanates on the other side, as well as precursors thereof (polyisocyanates, polyols or prepolymers).

8. Polyamides and copolyamides which are derived from diamines and dicarboxylic acids and/or from aminocarboxylic acids or the corresponding lactams, such as polyamide 4, polyamide 6, polyamide 6/6, 6/10, 6/9, 6/12 and 4/6, polyamide 11, polyamide 12, aromatic polyamides obtained by condensation of m-xylene, diamine and adipic acid; polyamides prepared from hexamethylene diamine and isophthalic or/and terephthalic acid and optionally an elastomer as modifier, for example poly-2,4,4,-trimethylhexamethylene terphthalamide or poly-m-phenylene isophthalamide. Further copolymers of the aforementioned polyamides with polyolefins, olefin copolymers, ionomers or chemically bonded or grafted elastomers; or with polyethers, such as, for instance, with polyethylene glycol, polypropylene glycol or polytetra- methlylene glycols. Polyamides or copolyamides modified with EPDM or ABS. Polyamides condensed during processing (RIM-polyamide systems).

9. Polyesters which are derived from dicarboxylic acids and diols and/or from hydroxycarboxylic acids or the corresponding lactones, such as polyethylene terephthalate, polybutylene terephthalate, poly-1,4-dimethylol-cyclohexane terephthalate, poly-[2,2,-)4-hydroxyphenyl)-propane] terephthalate and polyhydroxybenzoates as well as blockcopolyether-esters derived from polyethers having hydroxyl end groups.

10. Polycarbonates and polyester-carbonates.

11. Crosslinkable epoxide resins which are derived from polyepoxides, for example from bis-glycidyl ethers or from cycloaliphatic di-epoxides.

The polymer is preferably polystyrene, especially high-impact polystyrene, an acrylonitrile-butadiene-styrene polymer optionally blended with a polycarbonate, blend of polyphenylene oxide with high-impact polystyrene, a polyester, a polyamide or an epoxy resin.

The proportion by weight of flame retardant compound of formula I is conveniently within the range of from 0.1 to 50% by weight, more preferably from 0.5 to 30% by weight of the polymer.

The compositions of the invention may also contain other conventional ingredients, such as heat stabilisers, light stabilisers, ultra-violet light absorbers, anti-oxidants, anti-static agents, preservatives, adhesion promotors, fillers, fibre reinforcement, pigments, lubricants, blowing agents, fungicides, plasticizers, processing aids, anti-dripping agents e.g. fluorinated polyolefins such as polytetrafluoroethylenes, other fire-retardant additives and smoke suppressants.

The flame retardant additive of formula I is preferably used in conjunction with a further flame retardant.

Examples of such further flame retardants are the oxides, sulphides, sulphates, halides, oxyhalides and borates of tin, iron, molybdenum, zinc and antimony e.g. tin oxide, iron oxide, molybdenum oxide, zinc oxide, antimony oxide and zinc borate; metal stannates e.g. magnesium calcium and zinc stannates phosphorus-containing esters and salts e.g. triaryphosphates, alkylarylphosphates and ammonium polyphosphates; halogen-, especially bromine- and chlorine-containing compounds e.g. decarbromodiphenylether, hexachlorocyclopentadiene, brominated polystyrene, halogenoalkyl phosphates and -phosphonate esters; metal hydrates especially hydrated alumina; and the dehydropolymers of compounds of mono-, di- or tri-substituted benzene, the substituents being a C5- and/or C6-cycloaliphatic hydrocarbon, especially 1,1 -diphenylbicyclohexyl or 1,1 -diphenylbicyclopentyl, as disclosed in DE-PS 2525697. The preferred further flame retardant is antimony oxide.

The further flame retardant is conveniently employed in the composition of the present invention in an amount within the range of from 1 to 15%, especially from 2 to 10% by weight, based on the weight of the polymer.

The following Examples further illustrate the present invention.

## Example 1

29.4 parts of pyromellitic dianhydride (PMDA), 175.4g of Tribromoneopentyl alcohol (TBNPA) and 180 millilitres of toluene are heated to reflux under a nitrogen atmosphere. 27 millilitres of concentrated sulphuric acid are added and the reaction mixture is heated under reflux for a further 4 hours during which time 24.7 millilitres of water are distilled from the reaction. The reaction mixture is concentrated under reduced pressure and the resulting solid product is triturated with 300 millilitres of methanol. The solid product is filtered, washed with a further 300 millilitres of methanol and dried at 70°C under vacuum to give 175.7g of tetrakis (tribromoneopentyl) pyromellitate having melting point > 200°C.

## Example 2

Using the procedure outlined in Example 1, 37.8g of hexabromodiphenyl -4,4-dianhydride (HBDPDA) 64.1g of tribromoneopentylalcohol (TBNPA) 96 millilitres of toluene and 10 millilitres of concentrated

sulphine acid afforded 40.9g of tetrakis (tribromoneopentyl) hexabromodiphenyl 3,3',4,4'-tetracarboxylic acid having melting point > 200 ° C.

The HBDPDA is obtained as follows:

In a 1 litre, 3 necked flask fitted with condenser, temperature probe, bromine addition funnel and magnetic stirrer bar, is placed 75.24g DPDA, 1g iodine, 1g iron, and 590ml oleum (30% $SO_3$). The solution is warmed to reflux and 136.58g $Br_2$ is weighed into the dropping funnel. Bromine is added over 5 hours keeping excess refluxing vapor to a minimum. The reaction mixture is cooled and filtered. The solid product is washed with $H_2SO_4$, then re-slurried in 1 litre water and the pH adjusted to 14 with 100ml of 50% NaOH solution. The suspension is filtered, 500ml of ethyl acetate is added to the filtrate and the pH reduced to 0.2 with 150ml concentrated HCl. This mixture is stirred for 2 hours at 35 ° C. The aqueous phase is separated from ethyl acetate phase and the latter is washed with 1N HCl, then $H_2O$. Ethyl acetate is evaporated under vacuum to yield a white powder which is dried in vacuo at 150 ° C to give 130g product (66% yield).

Examples 3 to 9

The products from Examples 1 and 2 with the addition of antimony oxide are compounded into HIPS (high impact polystyrene poly-grade, 464 from Huntsman) and ABS (acrylonitrile-butadiene-styrene polymer-Magnum 3513 from DOW) in the proportions given in the table below, based on 100 parts by weight of polymer.

Compounding is performed using a torque rheometer at a temperature of 180 ° C and a mixing time of 8 minutes. Test specimens are prepared from the compounded product by compression molding at 200 ° C.

The Limiting Oxygen Index (L.O.I) of the specimen is measured according to the method of ASTM 2863-87 standard and the flammability of the specimen is tested according to the method of "Test for Flammability of Plastics Materials" Underwriter Laboratories Subject 94 (UL94) standard at a specimen thickness of 3.2mm. Results are shown for controls also, wherein the products from Example 1 and 2 and the antimony oxide were excluded.

In the UL94 standard, 94V-O is the highest rating, followed by 94V-1 and 94V-2 in descending order. "Freely burned" indicates failure to meet any of these ratings.

| | Product of Example 1 Parts by Weight | Product of Example 2 Parts by Weight | Antimony Oxide Parts by Weight | Polymer type | UL94 (3.2 mm) | L.O.I (%) |
|---|---|---|---|---|---|---|
| CONTROL 1 | - | - | - | ABS | Freely burned | 18.0 |
| CONTROL 2 | - | - | - | HIPS | Freely burned | 17.6 |
| 3 | 11.6 | - | 2.5 | ABS | 94V-2 | 22.0 |
| 4 | 19.3 | - | 4.16 | ABS | 94V-2 | 23.5 |
| 5 | 11.6 | - | 2.5 | HIPS | 94V-2 | 22.8 |
| 6 | 19.3 | - | 4.16 | HIPS | 94V.2 | 24.5 |
| 7 | - | 10.6 | 2.5 | ABS | 94V-2 | 22.4 |
| 8 | - | 10.6 | 2.5 | HIPS | 94V-2 | 20.8 |
| 9 | - | 17.66 | 4.16 | HIPS | 94V-0 | 22.7 |

**Claims**

**1.** A compound characterized by the formula I

$$Xn - Ar - [CO_2CH_2C(CH_2Br)_3]_m \qquad (I)$$

where Ar represents phenyl, naphthyl, or two aryl rings either directly connected or linked via $-CH_2-$.

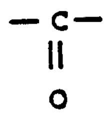 . 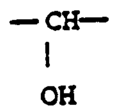 .

-O-.

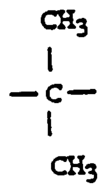 .

-CH = CH-. -CHBr- CHBr-. -S-. or

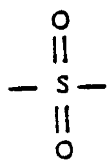

X is Cl or Br, m is 3 or 4; and n is 0 to 6; there being two of the ester groups in the ortho position to each other.

**2.** A compound as claimed in claim 1 characterized in that X is Br.

**3.** Tetrakis (tribromoneopentyl) pyromellitate.

**4.** Tetrakis (tribromoneopentyl) hexabromodiphenyl 3,3'4,4'-tetracarboxylic acid.

**5.** A flame retarded plastics or rubber composition characterized in that the flame retardant is a compound as claimed in claim 1 to 4.

**6.** A composition as claimed in claim 5 in which the plastics or rubber component is an ABS copolymer, high impact polystyrene or other engineering plastics material.

**7.** A composition as claimed in claim 5 or 6 characterized in that the amount of compound of formula I is from 0.1 to 50% by weight, based on the weight of the polymer.

**8.** A composition as claimed in claim 7 characterized in that the amount of compound of formula I is from 0.5 to 30% by weight.

**9.** A composition as claimed in any one of claims 5 to 8 characterized in that it also contains another flame retardant.

**10.** A composition as claimed in claim 9 characterized in that the other flame retardant is antimony oxide.

**11.** A composition as claimed in claim 9 or 10 characterized in that the amount of either flame retardant is from 1 to 15% by weight based on the weight of the polymer.

**12.** A composition as claimed in claim 11 characterized in that the amount of other flame retardant is from 2 to 10% by weight.